Europäisches Patentamt

European Patent Office

Office européen des brevets

(11) Veröffentlichungsnummer: **0 067 933**
**B1**

(19)

(12) **EUROPÄISCHE PATENTSCHRIFT**

(45) Veröffentlichungstag der Patentschrift :
20.02.85

(21) Anmeldenummer : 82103004.6

(22) Anmeldetag : 08.04.82

(51) Int. Cl.⁴ : **G 01 N 24/06**, **H 01 F 7/20**,
**A 61 B 5/05**

(54) **Elektromagnet für die NMR-Tomographie.**

(30) Priorität : 13.06.81 DE 3123493

(43) Veröffentlichungstag der Anmeldung :
29.12.82 Patentblatt 82/52

(45) Bekanntmachung des Hinweises auf die Patenterteilung : 20.02.85 Patentblatt 85/08

(84) Benannte Vertragsstaaten :
AT BE CH FR GB IT LI LU NL SE

(56) Entgegenhaltungen :
EP-A- 0 011 335
DE-A- 1 946 059
FR-A- 2 295 542
GB-A- 2 027 208
US-A- 3 564 398
ELECTRO/78 CONFERENCE RECORD, 23.-25. Mai
1978, Seiten 30/2 1-15, Boston, USA CHING-MING LAI
et al.: "Nuclear magnetic resonance zeugmatography for medical imaging"
Nachr. Chem. Tech. Lab. 28 (1980) Nr. 12 S. 860-865
J. Appl. Phys. vol. 40 Nr. 8 (1969) S. 3171-3179

(73) Patentinhaber : Bruker Analytische Messtechnik
GmbH
Silberstreifen
D-7512 Rheinstetten-Forchheim (DE)

(72) Erfinder : Knüttel, Bertold
Baumgartenstrasse 5
D-7512 Rheinstetten 2 (DE)
Erfinder : Lauklen, Rudi Günter Prof. Dr.
Silberstreifen
D-7512 Rheinstetten-4 (DE)

(74) Vertreter : Patentanwälte Kohler - Schwindling -
Späth
Hohentwielstrasse 41
D-7000 Stuttgart 1 (DE)

## Beschreibung

Die Erfindung betrifft einen Elektromagneten zur Erzeugung des statischen Magnetfeldes für die NMR-Tomographie der im Oberbegriff des Anspruchs 1 genannten Art. Ein derartiger Magnet ist beispielsweise bekannt aus Electro/78 Conference Record 23-25 Mai 1978 Boston USA, Seiten 30/2, 1-15.

Es hat sich herausgestellt, daß die NMR-Spektroskopie zur Erzeugung von Querschnittsbildern organischer Körper und insbesondere zur medizinischen Untersuchung des menschlichen Körpers geeignet ist, und es sind bereits entsprechende Versuche gemacht und hierfür geeignete Geräte entwickelt worden (Nachr. Chem. Tech. Lab. 28 (1980), Nr. 12, Seiten 860 bis 865). Diese Erzeugung von Querschnittsbildern wird NMR-Tomographie, gelegentlich auch NMR-Zeugmatographie oder NMR-Imaging genannt. Ein erhebliches Problem bei der NMR-Tomographie besteht in der Erzeugung eines homogenen statischen Magnetfeldes von den Dimensionen des zu untersuchenden Körperabschnittes. Homogene Magnetfelder einer Größe, wie sie insbesondere zur Untersuchung des menschlichen Körpers benötigt werden, dürften mit vertretbarem Aufwand nur im Inneren von Stromspulen erzeugbar sein, da Elektro-Eisenmagnete mit in entsprechend großem Abstand angeordneten Polschuhen ausreichender Größe viel zu aufwendig und insbesondere viel zu schwer würden. So ist in der oben angegebenen Literaturstelle ein Elektromagnet für die NMR-Tomographie angegeben, die vier wassergekühlte Stromspulen in einer sogenannten Doppel-Helmholtz-Anordnung umfaßt.

Die Erzeugung von homogenen Magnetfeldern mittels solcher Teilspulen-Anordnungen erfordert eine sehr genaue Bemessung und Justierung der einzelnen Spulen, um die hohen Homogenitätsanforderungen zu erfüllen und störende Feldkomponenten höherer Ordnung durch Kompensation zu eliminieren. Schon kleine Lageänderungen können die Kompensation und damit die Homogenität des Magnetfeldes stören. Solche Lageänderungen treten leicht infolge unterschiedlicher Wärmeausdehnung von Komponenten des die einzelnen Spulen tragenden Gestelles auf. Daher wird beim Einsatz solcher Spulenanordnungen in Laboratorien usw. auf eine gute Konstanz der Raumtemperatur geachtet. Diese Störeinflüsse sind natürlich umso größer, je größer die Spulenanordnungen sind. Für die NMR-Tomographie werden sehr große Spulenanordnungen benötigt, wenn der menschliche Körper oder auch tierische Körper untersucht werden sollen und daher in den von den Spulen umschlossenen Raum hineinpassen müssen. Der Mindest-Innendurchmesser solcher, für die Untersuchung des menschlichen Körpers bestimmter Spulenanordnungen dürfte etwa 650 mm betragen.

Eine weitere Fehlerquelle ist der Einfluß äußerer Magnetfelder. Es besteht zwar grundsätzlich die Möglichkeit, die Räume, in denen solche Apparaturen stehen, gegen äußere Magnetfelder abzuschirmen, jedoch bedeutet eine solche Abschirmung einen großen Aufwand.

Ein weiteres Problem besteht bei der NMR-Tomographie darin, daß die zur Erzeugung des HF-Magnetfeldes benötigten Spulen ebenfalls einen sehr großen Durchmesser haben müssen und daher in erheblichem Maße als strahlende Antennen wirken. Auch hierdurch können wiederum erhebliche Störungen entstehen, insbesondere wenn sich in dem Arbeitsraum stehende Wellen ausbilden, die örtlich sehr stark schwankende Feldstärken des HF-Feldes zur Folge haben. Außerdem ist hiermit ein Energieverlust verbunden, und es wird die Empfindlichkeit des Gerätes beeinflußt.

Neben den störenden Rückwirkungen auf die NMR-Tomographie kann das Abstrahlen eines HF-Feldes sich auch auf elektromagnetische Geräte auswirken, die sich im gleichen Raum befinden wie die Spulenanordnung. Hiervon betroffen sind insbesondere Peripherie-Geräte, die zur NMR-Tomographie benötigt werden, wie beispielsweise Plattenspeicher, Floppy Disks usw.

Bei den bekannten Elektromagneten ist die Strombelastung der Spulen und damit der Leistungsbedarf extrem hoch. In jedem Fall sind Kühlungsmaßnahmen in einer Größenordnung erforderlich, die nicht nur einen erheblichen Aufwand erfordern, sondern auch Anlaß zu Temperatur-Gradienten geben können, die wiederum zu unterschiedlichen Wärmeausdehnungen und damit zu einer ungünstigen Veränderung der geometrischen Zuordnung der einzelnen Bauelemente eines solchen Elektromagneten führen, die, wie oben angegeben, Störungen der Homogenität des Magnetfeldes zur Folge hat.

Alle diese Probleme lassen es fast unmöglich erscheinen, einen Elektromagneten zu schaffen, der gegen Störungen so unempfindlich ist, daß er in der normalen Röntgenarzt-Praxis, in diagnostischen Abteilungen von Krankenhäusern oder sonstigen zoologischen oder biologischen Instituten ohne besondere Schwierigkeiten eingesetzt werden kann und der zugleich einfach im Aufbau und in der Herstellung ist und auch für die praktische Anwendung ausreichend kleine Dimensionen und einen ausreichend kleinen Strombedarf hat. Der Erfindung liegt die Aufgabe zugrunde, einen solchen Elektromagneten zu schaffen.

Diese Aufgabe wird nach der Erfindung durch den im Anspruch 1 definierten Elektromagneten gelöst.

Bei dem erfindungsgemäßen Elektromagneten wird von einer vergleichsweise unkomplizierten Spulenanordnung in Verbindung mit einem Zylindermantel aus Eisen Gebrauch gemacht, der die Gestalt des von den Spulen erzeugten Magnetfeldes erheblich beeinflußt und bei der Berechnung der Spule zu berücksichtigen ist. Da bisher die für die NMR-Tomographie verwendeten Elektromagnete allgemein in Form von Luftspulen hergestellt worden sind, könnte die Beeinflussung des Magnetfeldes, welche die Verwendung des Zylindermantels aus Eisen zur Folge hat, zunächst als ungünstig angesehen werden. Der Zylindermantel hat jedoch den Vorteil, daß die dadurch erzielte Rückführung des Magnetflusses eine erhebliche Senkung der Leistungsaufnahme des Elektromagneten zur Folge hat. Weiterhin hat die Anwendung des Zylindermantels aus Eisen sowohl eine Abschirmung gegen äußere Magnetfelder als auch gegen eine äußere, elektromagnetische Störstrahlung zur Folge. Weiterhin wird nicht nur das von dem Elektromagneten selbst erzeugte Magnetfeld auf den vom Zylindermantel begrenzten Raum beschränkt, sondern auch das bei der NMR-Tomographie erzeugte HF-Feld an einem Abstrahlen nach außen gehindert.

Der Hauptvorteil der Anwendung eines stabilen Zylindermantels aus Eisen besteht jedoch darin, daß ein mechanisch äußerst stabiler Aufbau erzielt wird, der zudem noch sehr leicht zu fertigen ist. Die hohe Stabilität ergibt sich aus der absoluten Symmetrie des Zylindermantels sowohl in bezug auf seine Achse als auch in bezug auf die mittlere Querschnittsebene. Diese Symmetrie bleibt auch bei Temperaturänderungen erhalten. Die Folge hiervon ist, daß die bei Temperaturänderungen auftretenden Dimensionsänderungen keine nennenswerte Störung der Homogenität des Magnetfeldes zur Folge haben. Daher kann auf aufwendige Maßnahmen der Temperaturkompensation oder der Temperatur-Konstanthaltung verzichtet werden. Gleichzeitig hat der verminderte Leistungsbedarf neben der Verringerung der Betriebskosten eine verminderte Erwärmung der Spulen zur Folge, so daß sich die notwendigen Kühlungsmaßnahmen vereinfachen. Voraussetzung für die hohe mechanische Stabilität sowie die Verminderung des Leistungsbedarfes und zugleich die gute Abschirmung ist die Verwendung eines Zylindermantels erheblicher Dicke, wie sie für einfache Abschirmmaßnahmen nicht erforderlich wäre.

Bei dem erfindungsgemäßen Elektromagneten kann von Spulenanordnungen Gebrauch gemacht werden, wie sie grundsätzlich aus Journal of Applied Physics, Band 40, Nr. 8 (Juli 1969), Seiten 3171 bis 3179, bekannt sind. Allerdings bestehen auch diese bekannten Anordnungen ausschließlich aus Luftspulen, deren Gestalt im Hinblick auf die Anwendung des Zylindermantels aus Eisen modifiziert werden muß. Die Dimensionierung läßt sich unter Verwendung von numerischen Rechenverfahren an Rechenanlagen durchführen. Dabei ist es möglich, eine Feldspule zu verwenden, die aus mindestens zwei, in definiertem Abstand voneinander angeordneten Teilspulen besteht. An mindestens einem Ende der Feldspule bzw. der sie bildenden Teilspulen ist eine zur Feld- bzw. Teilspule konzentrische Korrekturspule angeordnet, wobei die Korrekturspule die Feld- bzw. Teilspule vorzugsweise konzentrisch umgibt.

Bei einer bevorzugten Ausführungsform der Erfindung sind die Spulen auf Spulenkörpern angeordnet, die mittels Distanzscheiben an der Innenseite des Zylindermantels befestigt und zentriert sind. Auf diese Weise ist eine genau koaxiale und auch thermisch symmetrische Anordnung der Spulen zu dem Zylindermantel möglich.

Die Wirksamkeit des Zylindermantels als Abschirmung und als magnetische Rückführung kann noch dadurch verbessert werden, daß in weiterer Ausgestaltung der Erfindung am Ende des Zylindermantels die Stirnflächen der Spulen überdeckende Ringscheiben aus Eisen angebracht sind. Es versteht sich, daß auch diese Ringscheiben ebenso wie der Zylindermantel eine erhebliche Dicke haben müssen. Weiterhin lassen sich die Ringscheiben mit zur Kompensation von Inhomogenitäten des Magnetfeldes ausnutzen.

Sowohl zur Verbesserung der Homogenität des Magnetfeldes als auch zur Verbesserung der Abschirmung kann es zweckmäßig sein, daß sich an die Außenseiten der Ringscheibe noch Rohrstutzen aus Eisen anschließen, deren Durchmesser höchstens gleich dem Durchmesser der Zylinderspulen ist. Es versteht sich, daß es sich auch bei diesen Rohrstutzen um sorgfältig bemessene rotationssymmetrische Bauteile handelt, deren Ausbildung bei der Bemessung der Spulen zu berücksichtigen ist.

Soweit es die beabsichtigte Verwendung des Elektromagneten zuläßt, könnte der Zylindermantel an wenigstens einem Ende auch durch einen Eisendeckel gänzlich verschlossen sein. Auch hier kann durch die Lage und Form des Eisendeckels das erzeugte Magnetfeld in erheblichem Maße beeinflußt werden, um dessen Homogenität zu fördern. Tatsächlich entsteht durch die Anbringung des Eisendeckels eine Spiegelung des Magnetfeldes an dessen Fläche, was eine scheinbare Verdoppelung der Spulenlänge zur Folge hat, woraus wiederum eine erhöhte Homogenität bei reduziertem Leistungsbedarf resultiert. Bei einer bevorzugten Ausführungsform der Erfindung ist der Eisendeckel gewölbt, wodurch eine zur Erhöhung der Homogenität beitragende Fokussierung der Feldlinien in dem an den Deckel angrenzenden Bereich erzielt wird.

Erfindungsgemäße Elektromagnete, die einen wenigstens an einem Ende offenen Zylindermantel aufweisen, sind im wesentlichen für Untersuchungen geeignet, bei denen die Längsachse des zu untersuchenden Körpers mit der Längsachse des Elektromagneten zusammenfallen soll und daher der Körper von einem Ende her in Richtung seiner Längsachse in den Elektromagneten eingeführt werden kann. Dabei ist eine stehende oder liegende Lage des Elektromagneten möglich.

Insbesondere für Untersuchungen, bei denen die Längsachse des zu untersuchenden Körpers senkrecht zur Längsachse des Elektromagneten stehen soll, ist eine spezielle Ausführungsform des erfindungsgemäßen Elektromagneten zweckmäßig, bei welcher der Zylindermantel an beiden Enden durch einen Deckel verschlossen ist und eine zu seiner mittleren Querebene symmetrische Öffnung aufweist, die das Einführen des zu untersuchenden Körpers quer zur Längsachse des Elektromagneten gestattet. Bei dieser Ausführungsform bestehen die Feldspulen aus zu beiden Seiten der Öffnung angeordneten, symmetrischen Teilspulen, die vorzugsweise jeweils an ihrem der Öffnung zugewandten Ende mit einer Korrekturspule versehen sind.

Eine andere, für spezielle Untersuchungen besonders geeignete Ausführungsform des Elektromagneten ist dadurch gekennzeichnet, daß der an einem Ende durch einen Deckel verschlossene Zylindermantel am anderen Ende über die Feldspule hinaus verlängert ist, in dem über die Feldspule überstehenden Abschnitt eine bis zu seinem Ende reichende Öffnung aufweist und an diesem Ende mit einem entfernbaren Deckel versehen ist. Bei dieser Ausführungsform kann der entfernbare Deckel mit besonderem Vorteil als Sitzplatte ausgebildet und der Zylindermantel mit der Spulenanordnung mit senkrechter Achse in Vertikalrichtung beweglich angeordnet sein. Diese Ausführungsform der Erfindung ermöglicht demnach Untersuchungen am Oberkörper eines sitzenden Menschen in einem Elektromagneten, dessen Abschirmung nahezu völlig geschlossen ist.

Wie erwähnt, kann der Elektromagnet außer einer Feldspule auch Korrekturspulen umfassen, die innerhalb oder außerhalb der Feldspule konzentrisch angeordnet sind (koaxiale Shimsysteme) und zur Verbesserung der Homogenität, aber auch zur Erzeugung spezieller Gradienten des Magnetfeldes dienen. In der NMR-Tomographie ist es oftmals erforderlich, die Gradienten zu schalten. Um dadurch bedingte Wirbelströme in dem Zylindermantel möglichst klein zu halten, kann der Zylindermantel in weiterer Ausgestaltung der Erfindung in Längsrichtung ein- oder mehrfach geschlitzt sein. Es können dann wenigstens keine geschlossenen Ströme in Umfangsrichtung des Zylindermantels entstehen.

Die Erfindung wird im folgenden anhand der in der Zeichnung dargestellten Ausführungsbeispiele näher beschrieben und erläutert. Die der Beschreibung und der Zeichnung zu entnehmenden Merkmale können bei anderen Ausführungsformen der Erfindung einzeln für sich oder zu mehreren in beliebiger Kombination Anwendung finden. Es zeigen :

Figur 1 einen Längsschnitt durch eine erste Ausführungsform eines Elektromagneten nach der Erfindung,

Figur 2 einen Längsschnitt durch eine zweite Ausführungsform eines Elektromagneten nach der Erfindung und

Figur 3 bis 6 schematische Darstellungen unterschiedlicher Elektromagnete nach der Erfindung in ihrer Anwendung bei der NMR-Tomographie am menschlichen Körper.

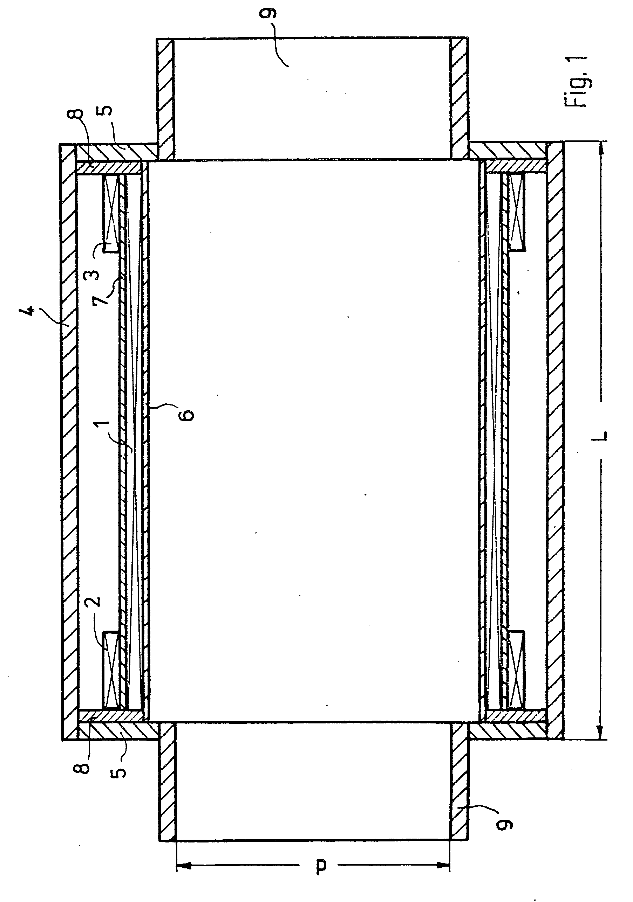

Der in Figur 1 dargestellte Elektromagnet weist eine zylindrische Feldspule 1 und an deren Enden angeordnete Korrekturspulen 2 und 3 auf, welche die Feldspule 1 konzentrisch umgeben. Die Spulen 1 bis 3 sind mit Abstand von einem Zylindermantel 4 aus weichem Eisen umgeben, dessen Länge L etwas größer ist als diejenige der Zylinderspule 1. Die Spulen 1 bis 3 sind auf Spulenkörpern 6 und 7 gewickelt, die mittels Distanzschscheiben 8 gehaltert und gleichzeitig gegenüber dem Zylindermantel 4 zentriert sind. Gegebenenfalls könnten auch die Ringscheiben 5 die Halte- und Zentrierfunktion übernehmen. Die Spulenkörper 6 und 7 sowie die Distanzscheiben 8 sind aus unmagnetischen Materialien wie etwa Aluminium oder Rotguß gefertigt. In die Öffnungen der Ringscheibe 5 sind weiterhin Rohrstutzen 9 eingesetzt, die ebenfalls aus Eisen bestehen und deren Durchmesser etwas geringer ist als der Durchmesser der Zylinderspule 1.

Länge L des Zylindermantels 4 und der Innendurchmesser d der Rohrstutzen 9 sind im wesentlichen durch die Abmessungen des zu untersuchenden Körpers und die geforderte Homogenität des Magnetfeldes bestimmt. Im Hinblick auf die vorgesehenen Dimensionen bei der Untersuchung des menschlichen Körpers ist der Platzbedarf des Elektromagneten nach Figur 1 vergleichsweise gering und jedenfalls bedeutend geringer als der Platzbedarf der bekannten Doppel-Helmholtz-Anordnung.

Der starkwandige Zylindermantel 4 aus Eisen gewährleistet in Verbindung mit den Distanzscheiben 8 eine sehr sichere und genau zentrische Halterung der Spulen 1 bis 3, die auch bei Temperaturschwankungen mit den damit verbundenen Dimensionsänderungen erhalten bleibt, weil der Aufbau vollkommen symmetrisch ist. Als Folge davon bleibt auch die Homogenität des Magnetfeldes in dem interessierenden Bereich erhalten, so daß Kompensationsmaßnahmen nicht erforderlich sind. Die Rückführung des äußeren Magnetfeldes durch den aus Eisen bestehenden Zylindermantel 4 und die Ringscheiben 5 hat eine beträchtliche Verminderung der Leistungsaufnahme zur Folge, was sowohl für die Betriebskosten als auch für die Stabilität des Elektromagneten von Bedeutung ist. Zugleich bietet die Gestaltung der Eisenhülle, insbesondere die Dimensionierung der Ringscheiben 5 und der Rohrstutzen 9 eine die Wirkung der Korrekturspulen 2, 3 ergänzende, zusätzliche Möglichkeiten zur Homogenisierung des Magnetfeldes. Weiterhin bewirkt der sehr dicke Eisenmantel sowohl eine hervorragende Abschirmung gegen äußere Magnetfelder und eine äußere elektromagnetische Störstrahlung als auch gegen ein Abstrahlen des bei der NMR-Tomographie innerhalb der Abschirmung erzeugten HF-Feldes. Zugleich hat der Elektromagnet einen sehr einfachen Aufbau, der eine kostengünstige Fertigung trotz Einhaltung engster Toleranzen ermöglicht. Die notwendige Kühlung kann in bekannter Weise durch ein Kühlmedium erfolgen, das entweder durch Kühleinrichtungen geleitet wird, die mit den Spulenkörpern 6 und 7

verbunden sind, oder aber direkt ein Hohlprofil durchfließt, das zum Wickeln der Spulen verwendet wurde.

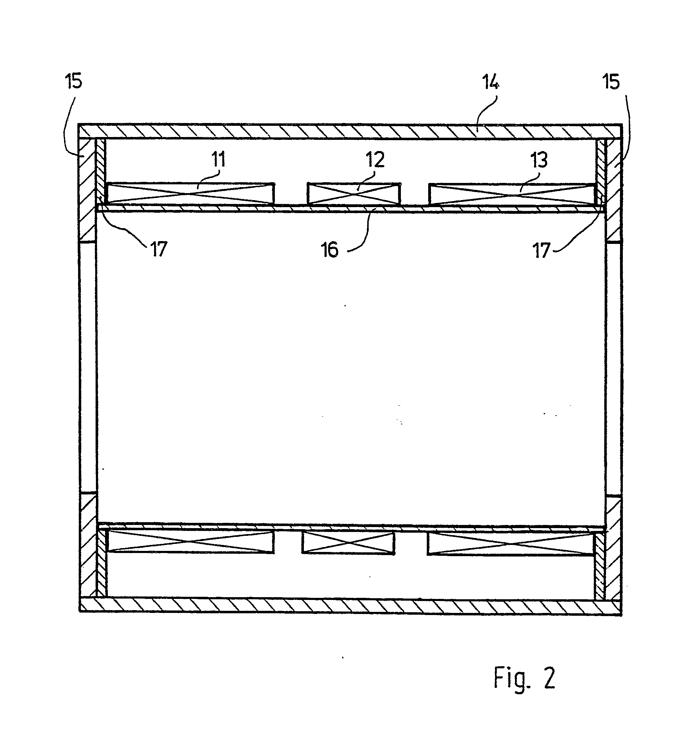

Bei dem in Figur 2 dargestellten Ausführungsbeispiel besteht die Feldspule aus drei Teilspulen 11, 12, 13, die auf einem gemeinsamen Spulenkörper 16 mit definiertem Abstand nebeneinander angeordnet sind. Der Spulenkörper 16 ist wiederum mittels Distanzscheiben 17 gegen den Zylindermantel 14 abgestützt und zentriert. Der Zylindermantel 14 und die Ringscheiben 15 bestehen aus dickwandigem Eisen und bilden sowohl eine Rückführung für das äußere Magnetfeld als auch eine magnetische und elektrische Abschirmung. Die einzelnen Teilspulen 11, 12 und 13 sowie deren Abstände sind in Verbindung mit der Dimensionierung des Zylindermantels 14 und der Ringscheiben 15 so bemessen, daß im Inneren der Spulenanordnung ein Magnetfeld hoher Homogenität herrscht. Auch hier ist wieder ein sehr stabiler und symmetrischer Aufbau vorhanden, so daß der Elektromagnet nach Figur 2 die gleichen vorteilhaften Eigenschaften aufweist wie der Elektromagnet nach Figur 1. Die geänderte Spulenanordnung erlaubt eine geringere Baulänge auf Kosten eines etwas größeren Strombedarfes.

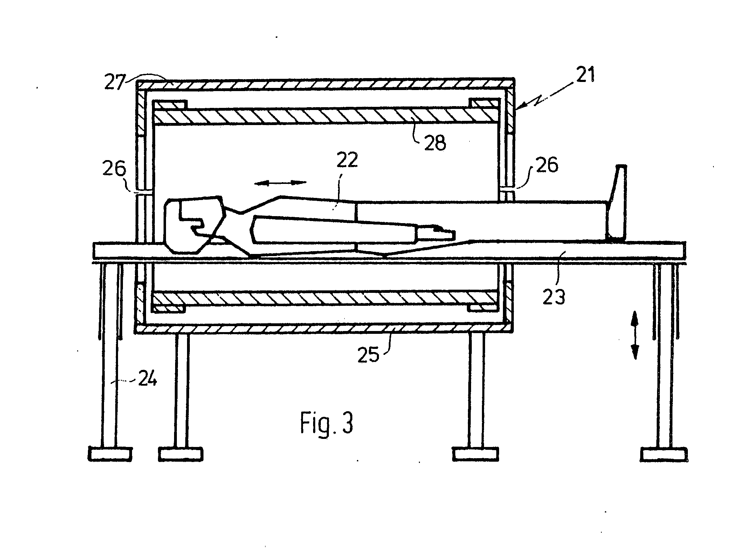

Fig. 3 zeigt die Anwendung eines Elektromagneten der in Fig. 1 dargestellten Art zur Untersuchung des menschlichen Körpers. Wie ersichtlich, hat der Elektromagnet 21 so große Abmessungen, daß er wesentliche Teile eines menschlichen Körpers 22 aufzunehmen vermag. Bei der dargestellten Einrichtung ist der Elektromagnet 21 mit horizontaler Achse angeordnet und es befindet sich der menschliche Körper 22 auf einem den Elektromagneten durchdringenden Tisch 23, der mittels Teleskopbeinen 24 in der Höhe verstellbar ist. Außerdem kann eine Einrichtung zum Verschieben des Tisches 23 in Richtung der Längsachse des Magneten 21 vorgesehen werden. Bei diesem Ausführungsbeispiel ist der Zylindermantel 25 an zwei diametral gegenüberliegenden Stellen 26 geschlitzt, so daß die obere Hälfte 27 des Zylindermantels von der unteren abhebbar ist und die Erregerspule 28 und sonstige innerhalb des Zylindermantels angeordnete Einrichtungen leichter zugänglich werden. Weiterhin verhindern die Längsschlitze 26 das Entstehen von in Umfangsrichtung des Zylindermantels geschlossenen Wirbelströmen, wie sie insbesondere beim Zu- und Abschalten von Korrekturspulen entstehen können.

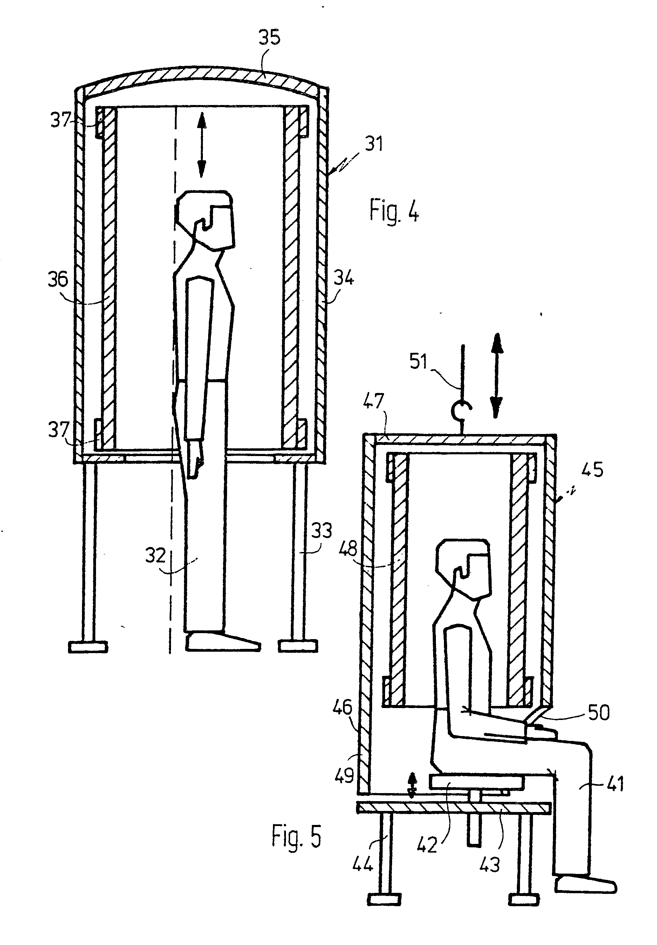

Wie Fig. 4 zeigt, ist es grundsätzlich möglich, einen Elektromagneten 31 nach der Erfindung auch mit senkrechter Achse anzuordnen, so daß der Elektromagnet den Oberkörper eines stehenden, zu untersuchenden Patienten 32 umschließt. Bei dieser Ausführungsform ist der Elektromagnet 31 an seinem unteren Ende auf Stützen 33 angeordnet, die gegebenenfalls teleskopisch ausfahrbar sein können, um den Magneten 31 anzuheben, damit der Patient sich ohne Mühe an den Untersuchungsort begeben kann, wonach der Elektromagnet 31 über den Patienten abgesenkt wird. Alternativ kann der Magnet mittels eines Seilzuges hochgezogen oder abgesenkt werden. Bei dieser Ausführungsform besteht kein Bedarf dafür, daß der Elektromagnet 31 an seinen beiden Enden offen ist. Daher ist in diesem Fall der aus Eisen bestehende Zylindermantel 34 des Magneten an seinem oberen Ende durch einen Deckel 35 verschlossen. Die Anordnung eines solchen Dekkels hat eine Spiegelung des Magnetfeldes an der Fläche dieses Deckels zur Folge, was zu einer scheinbaren Verdoppelung der Spulenlänge führt. Hierdurch ergibt sich eine Erhöhung der Homogenität bei reduziertem Leistungsbedarf. Dabei ist die Wölbung des Deckels 35 noch von besonderem Vorteil, weil sie eine Fokussierung der Feldlinien und infolgedessen eine erhöhte Homogenität des Feldes im oberen Drittel des Magneten zur Folge hat. Es versteht sich, daß auch hier die Wirkungen des Deckels 35 bei der Dimensionierung der Feld- und Korrekturspulen 36, 37 berücksichtigt werden muß.

Wenn der Patient bei der Untersuchung eine sitzende Haltung einnimmt, kann ein Elektromagnet Verwendung finden, der an seinen beiden Enden durch einen Eisendeckel verschlossen ist, wie es Fig. 5 zeigt. Wie aus Fig. 5 ersichtlich, ermöglicht diese Ausführungsform eines Elektromagneten, daß sich der Patient 41 auf einem Sitz 42 niederläßt, der sich an der Oberseite einer Eisenplatte 43 befindet, die mittels Füßen 44 am Boden abgestützt ist. Der mit vertikaler Achse angeordnete Elektromagnet 45 weist einen aus Eisen bestehenden Zylindermantel 46 auf, der an seinem oberen Ende durch einen Eisendeckel 47 verschlossen ist und einen über die Spulenanordnung 48 überstehenden Abschnitt 49 besitzt, der bei abgesenktem Elektromagneten 45 auf dem Rand der Eisenplatte 43 aufsitzt. In dem über die Spulenanordnung 48 überstehenden Abschnitt befindet sich eine bis zum Rand dieses Abschnittes reichende Öffnung 50, die groß genug ist, um die Beine des Patienten 41 passieren zu lassen. Der Elektromagnet 45 kann mittels eines Seilzuges 51 oder dergleichen von der Eisenplatte 43 abgehoben werden, um dem Patienten 41 einen guten Ein- und Ausstieg zu ermöglichen. Bei abgesenktem Elektromagneten 45 bildet die Eisenplatte 43 einen den Elektromagneten nach unten abschließenden Deckel.

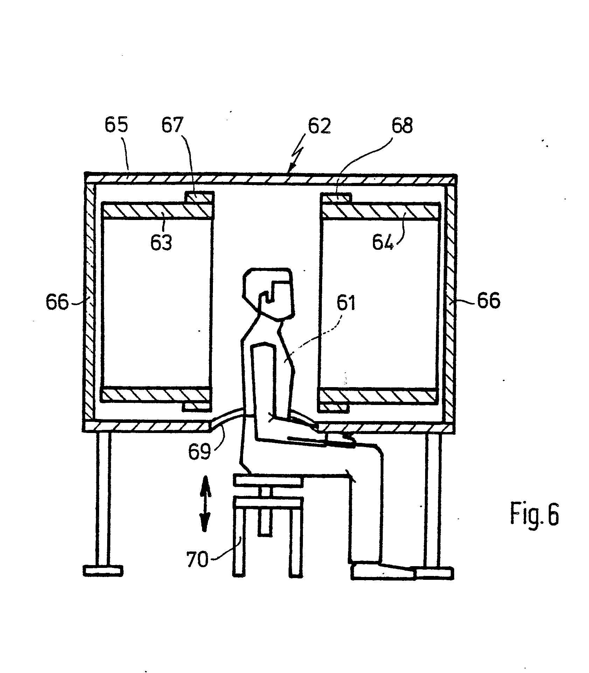

Während bei den bisher beschriebenen Ausführungsformen die Längsachse des Körpers des Patienten stets mit der Längsachse des Elektromagneten zusammenfiel, zeigt Fig. 6 eine Ausführungsform, bei welcher der Oberkörper 61 des Patienten in den Elektromagneten 62 quer zu dessen Längsachse hineinragt. Bei dieser Ausführungsform der Erfindung weist der Elektromagnet 62 eine Spulenanordnung auf, die aus zwei Teilspulen 63, 64 besteht, die in einem solchen Abstand voneinander angeordnet sind, daß zwischen ihnen der Oberkörper des Patienten 61 Platz hat. Sie sind von einem Zylindermantel 65 umgeben, der an seinen Enden durch Deckel 66 verschlossen ist. An ihren einander zugewandten Enden weisen die Teilspulen 63, 64 je eine sie konzentrisch umgebende Korrekturspule 67 bzw. 68 auf. Der aus Eisen bestehende Zylindermantel 65 weist an seiner Unterseite eine zu seiner Quermittelebene konzentrische Öffnung 69 auf, deren Durchmesser etwa dem Abstand zwischen den Teilspulen 63, 64 gleich ist und die demgemäß groß genug ist, um den Oberkörper des Patienten 61 passieren zu lassen. Der Elektromagnet 62 kann wiederum so angeordnet sein, daß er über den Oberkörper des Patienten abgesenkt und von diesem wieder abgehohen werden kann. Dabei kann der Patient eine sitzende Haltung einnehmen, wie dargestellt, oder aber auch stehend untersucht werden. Es wäre auch möglich, den Patienten 61 mittels eines in der Höhe verstellbaren Stuhles 70 von unten in den Elektromagneten 62 einzufahren.

Es versteht sich, daß die vorstehend behandelten Ausführungsbeispiele nur wenige von vielen möglichen Konfigurationen darstellen, deren spezielle Ausbildung sich nach dem Anwendungszweck, also insbesondere nach der Art des zu untersuchenden Körpers richtet.

## Ansprüche

1. Elektromagnet zur Erzeugung des statischen Magnetfeldes für die NMR-Tomographie mit einer Spulenanordnung, die aus mindestens einer kreiszylindrischen Feldspule (1 ; 11, 12, 13 ; 28 ; 36 ; 48 ; 63, 64) und mindestens einer zur Feldspule konzentrischen Korrekturspule (2, 3 ; 37 ; 67, 68) besteht und in einem von ihr definierten, zur Aufnahme des zu untersuchenden Körpers (22 ; 32 ; 41 ; 61) geeigneten und zugänglichen Innenraum ein wenigstens annähernd homogenes Magnetfeld erzeugt, dadurch gekennzeichnet, daß die Spulenanordnung von einem sie tragenden, stabilen Zylindermantel (4 ; 14 ; 25 ; 34 ; 46 ; 65) aus Eisen umgeben ist, dessen Einfluß auf die Homogenität des im Inneren der Spulenanordnung herrschenden Magnetfeldes durch die Dimensionierung der Feld- und Korrekturspulen kompensiert ist.

2. Elektromagnet nach Anspruch 1, dadurch gekennzeichnet, daß die Spulenanordnung mindestens zwei, in definiertem Abstand voneinander angeordnete Feldspulen (11, 12, 13 ; 63, 64) umfaßt.

3. Elektromagnet nach Anspruch 1 oder 2, dadurch gekennzeichnet, daß an mindestens einem Ende jeder Feldspule (1 ; 28 ; 36 ; 48 ; 63, 64) eine zur Feldspule konzentrische Korrekturspule (2, 3 ; 37 ; 67, 68) angeordnet ist.

4. Elektromagnet nach Anspruch 3, dadurch gekennzeichnet, daß die Korrekturspulen (2, 3 ; 37 ; 67, 68) die Feldspule (1) konzentrisch umgeben.

5. Elektromagnet nach einem der vorhergehenden Ansprüche, dadurch gekennzeichnet, daß die Feld- und Korrekturspulen auf Spulenkörpern (6, 7 ; 16) angeordnet sind, die mittels Distanzscheiben (8 ; 17) an der Innenseite des Zylindermantels (4 ; 14) befestigt und zentriert sind.

6. Elektromagnet nach einem der vorhergehenden Ansprüche, dadurch gekennzeichnet, daß an einem oder beiden Enden des Zylindermantels (4 ; 14) die Stirnflächen der Spulen überdeckende Ringscheiben (5 ; 15) aus Eisen angebracht sind.

7. Elektromagnet nach den Ansprüchen 5 und 6, dadurch gekennzeichnet, daß die Ringscheiben zugleich die die Spulenkörper mit dem Zylindermantel verbindenden Distanzstücke sind.

8. Elektromagnet nach Anspruch 6 oder 7, dadurch gekennzeichnet, daß sich an die Außenseite der Ringscheiben (5) Rohrstutzen (9) aus Eisen anschließen, deren Durchmesser (d) höchstens gleich dem Innendurchmesser der Feldspule (1) ist.

9. Elektromagnet nach einem der vorhergehenden Ansprüche, dadurch gekennzeichnet, daß der Zylindermantel (34 ; 46) an wenigstens einem Ende durch einen Eisendeckel (35 ; 47) verschlossen ist.

10. Elektromagnet nach Anspruch 9, dadurch gekennzeichnet, daß der Eisendeckel (35) gewölbt ist.

11. Elektromagnet nach Anspruch 9 oder 10, dadurch gekennzeichnet, daß der Zylindermantel (65) an beiden Enden durch je einen Deckel (66) verschlossen ist und eine zu seiner mittleren Querebene symmetrische Öffnung (69) aufweist und daß zwei Feldspulen (63, 64) zu beiden Seiten der Öffnung (69) symmetrisch zueinander angeordnet sind.

12. Elektromagnet nach Anspruch 11, dadurch gekennzeichnet, daß die Feldspulen (63, 64) jeweils an ihren einander zugewandten Enden mit einer Korrekturspule (67, 68) versehen sind.

13. Elektromagnet nach Anspruch 9 oder 10, dadurch gekennzeichnet, daß der an mindestens einem Ende durch einen Deckel (47) verschlossene Zylindermantel (46) am anderen Ende über die Feldspule (48) hinaus verlängert ist, in dem über die Feldspule überstehenden Abschnitt (49) eine bis zu seinem Ende reichende Öffnung (50) aufweist und an diesem Ende mit einem entfernbaren Eisendeckel (43) versehen ist.

14. Elektromagnet nach Anspruch 13, dadurch gekennzeichnet, daß der entfernbare Deckel (43) als Sitzplatte ausgebildet und der Zylindermantel (46) mit der Spulenanordnung mit senkrechter Achse in Vertikalrichtung beweglich angeordnet ist.

15. Elektromagnet nach einem der vorhergehenden Ansprüche, dadurch gekennzeichnet, daß der Zylindermantel (25) in Längsrichtung ein- oder mehrfach geschlitzt ist.

## Claims

1. Electromagnet for producing the static magnetic field for NMR-tomography with a coil arrangement which comprises at least one circular cylindrical field coil (1 ; 11, 12, 13 ; 28 ; 36 ;

48 ; 63, 64) and at least one correction coil (2, 3 ; 37 ; 67, 68) concentric to the field coil, and which produces an at least approximately homogeneous magnetic field in an inner space defined thereby and accessible to and adapted to receive the body (22 ; 32 ; 41 ; 61) to be investigated, characterised in that the coil arrangement is surrounded and carried by a stable cylindrical sleeve (4 ; 14 ; 25 ; 34 ; 46 ; 65) of iron the influence of which on the homogeneity of the magnetic field existing in the interior of the coil arrangement is compensated by the dimensioning of the field and correction coils.

2. Electromagnet according to claim 1, characterised in that the coil arrangement comprises at least two field coils (11, 12, 13 ; 63, 64) arranged at predetermined spacing from each other.

3. Electromagnet according to claim 1 or 2, characterised in that at least at one end of each field coil (1 ; 28 ; 36 ; 48 ; 63, 64) is arranged a correction coil (2, 3 ; 37 ; 67, 68) concentric to the field coil.

4. Electromagnet according to claim 3, characterised in that the correction coils (2, 3 ; 37 ; 67, 68) concentrically encircle the field coil (1).

5. Electromagnet according to one of the preceding claims, characterised in that the field and correction coils are arranged on coil bodies (6, 7 ; 16) which are fixed and centred internally of the cylindrical sleeve (4 ; 14) by means of spacers (8 ; 17).

6. Electromagnet according to one of the preceding claims, characterised in that at one or both ends of the cylindrical sleeve (4 ; 14) there are provided iron rings (5 ; 15) covering the end faces of the coils.

7. Electromagnet according to claims 5 and 6, characterised in that the rings also serve as the distance pieces connecting the coil bodies with the cylindrical sleeve.

8. Electromagnet according to claim 6 or 7, characterised in that to the outer surface of the rings (5) are connected iron stub pipes (9) whereof the diameter (d) is no greater than the internal diameter of the field coil (1).

9. Electromagnet according to one of the preceding claims, characterised in that the cylindrical sleeve (34 ; 46) is closed at least at one end by an iron cover (35 ; 47).

10. Electromagnet according to claim 9, characterised in that the iron cover (35) is arched.

11. Electromagnet according to claim 9 or 10, characterised in that the cylindrical sleeve (65) is closed at both ends by a respective cover (66) and has an opening (69) symmetrical with respect to its median transverse plane and that two field coils (63, 64) are arranged symmetrically with respect to each other at both sides of the opening (69).

12. Electromagnet according to claim 11, characterised in that the field coils (63, 64) are each provided at their opposed ends with a correction coil (67, 68).

13. Electromagnet according to claim 9 or 10, characterised in that the cylindrical sleeve (46) closed at least at one end by a cover (47) is extended at the other end beyond the field coil (48) and has in the section (49) extending beyond the field coil an opening (50) extending to the end thereof and is provided at this end with a removable iron cover (43).

14. Electromagnet according to claim 13, characterised in that the removable cover (43) is adapted as a sitting plate and the cylindrical sleeve (46) with the axis-vertical coil arrangement is movable in the vertical direction.

15. Electromagnet according to one of the preceding claims, characterised in that the cylindrical sleeve (25) is slotted once or several times in the longitudinal direction.

**Revendications**

1. Electroaimant pour la production du champ magnétique statique pour la tomographie RMN, avec un montage de bobines, constitué par au moins une bobine de champ cylindrique circulaire (1 ; 11, 12, 13 ; 28 ; 36 ; 42 ; 63, 64) et au moins une bobine de correction (2, 3 ; 37 ; 67, 68) concentrique à la bobine de champ, et produisant un champ magnétique au moins sensiblement homogène dans l'enceinte intérieure définie par ledit montage, accessible et recevant le corps à examiner (22 ; 32 ; 41 ; 61), ledit électroaimant étant caractérisé en ce que le montage de bobines est entouré par une enveloppe cylindrique en fer (4 ; 14 ; 25 ; 34 ; 46 ; 65) stable, le portant èt dont l'influence sur l'homogénéité du champ magnétique régnant à l'intérieur dudit montage est compensée par le dimensionnement des bobines de champ et de correction.

2. Electroaimant selon revendication 1, caractérisé en ce que le montage de bobines comprend au moins deux bobines de champ (11, 12, 13 ; 63, 64) disposées avec un écartement défini.

3. Electroaimant selon une des revendications 1 ou 2, caractérisé en ce qu'une bobine de correction (2, 3 ; 37 ; 67, 68) est disposée concentriquement sur une extrémité au moins de chaque bobine de champ (1 ; 28 ; 36 ; 48 ; 63, 64).

4. Electroaimant selon revendication 3, caractérisé en ce que les bobines de correction (2, 3 ; 37 ; 67, 68) entourent concentriquement la bobine de champ (1).

5. Electroaimant selon une quelconque des revendications 1 à 4, caractérisé en ce que les bobines de champ et de correction sont disposées sur des carcasses (6, 7 ; 16) que des bagues entretoises (8 ; 17) fixent et centrent sur la face intérieure de l'enveloppe cylindrique (4 ; 14).

6. Electroaimant selon une quelconque des revendications 1 à 5, caractérisé en ce que des bagues en fer (5 ; 15) sont disposées sur une des deux ou sur les deux extrémités de l'enveloppe cylindrique (4 ; 14) et recouvrent les faces frontales des bobines.

7. Electroaimant selon revendications 5 et 6, caractérisé en ce que les bagues constituent simultanément les entretoises reliant les carcasses à l'enveloppe cylindrique.

8. Electroaimant selon une des revendications 6 ou 7, caractérisé en ce que des tubulures (9) en fer font suite à la face extérieure des bagues (5) et présentent un diamètre (d) au maximum égal au diamètre intérieur de la bobine de champ (1).

9. Electroaimant selon une quelconque des revendications 1 à 8, caractérisé en ce qu'une extrémité au moins de l'enveloppe cylindrique (34 ; 46) est obturée par un couvercle en fer (35 ; 47).

10. Electroaimant selon revendication 9, caractérisé en ce que le couvercle en fer (35) est bombé.

11. Electroaimant selon une des revendications 9 ou 10, caractérisé en ce que l'enveloppe cylindrique (65) est obturée à chaque extrémité par un couvercle (66) et présente une ouverture (69) symétrique par rapport à son plan transversal médian ; et deux bobines de champ (63, 64) sont disposées symétriquement de part et d'autre de l'ouverture (69).

12. Electroaimant selon revendication 11, caractérisé en ce que les extrémités en regard des bobines de champ (63, 64) portent chacune une bobine de correction (67, 68).

13. Electroaimant selon une des revendications 9 ou 10, caractérisé en ce que l'enveloppe cylindrique (46) obturée d'un côté au moins par un couvercle (47) est prolongée de l'autre côté au-delà de la bobine de champ (48), la partie (49) en saillie sur la bobine de champ comportant une ouverture (50) s'étendant jusqu'à son extrémité, qui est munie d'un couvercle en fer (43) amovible.

14. Electroaimant selon revendication 13, caractérisé en ce que le couvercle amovible (43) est réalisé en siège et l'enveloppe cylindrique (46), avec le montage de bobines à axe vertical, est mobile verticalement.

15. Electroaimant selon une quelconque des revendications 1 à 14, caractérisé en ce que l'enveloppe cylindrique (25) présente une ou plusieurs fentes longitudinales.

Fig. 1

Fig. 2

Fig. 3

0 067 933

Fig. 4

Fig. 5

Fig. 6